# EUROPEAN PATENT SPECIFICATION

(11) **EP 2 670 324 B1**
(45) Date of publication and mention of the grant of the patent: **21.10.2020**
(21) Application number: 12741982.8
(22) Date of filing: 03.02.2012
(51) Int. Cl.: A61B 17/86, A61B 17/70, A61B 17/84

(54) **SEMI-RIGID SCREW ASSEMBLY**
HALBSTEIFE SCHRAUBENANORDNUNG
ENSEMBLE VIS SEMI-RIGIDE

(30) Priority: 05.02.2011 US 201161439858 P
(43) Date of publication of application: 11.12.2013
(73) Proprietor: Alphatec Spine, Inc., Carlsbad, CA 92008 (US)
(72) Inventor: BOULIANE, Martin, Carlsbad CA 92011 (US); PURCELL, Thomas, Del Mar CA 92014 (US)
(74) Representative: Haseltine Lake Kempner LLP
(86) International application number: PCT/US2012/023845
(87) International publication number: WO 2012/106646

(56) References cited:
- EP-A1- 2 022 423
- EP-A1- 2 153 786
- WO-A2-2006/089292
- WO-A2-2008/027853
- GB-A- 2 465 156
- US-A1- 2006 149 241
- US-A1- 2007 055 242
- US-A1- 2007 270 839
- US-A1- 2010 234 891

## Description

### FIELD

The present disclosure generally relates to the field of spinal orthopedics, and more particularly to a semi-rigid screw assembly that attaches to a vertebra.

### BACKGROUND

The spine is a flexible column formed of a plurality of bones called vertebrae. The vertebrae are hollow and stacked one upon the other, forming a strong hollow column for support of the cranium and trunk. The hollow core of the spine houses and protects the nerves of the spinal cord. The different vertebrae are connected to one another by means of articular processes and intervertebral, fibrocartilaginous bodies. Various spinal disorders may cause the spine to become misaligned, curved, and/or twisted. It is often necessary to surgically correct and stabilize spinal curvatures or to facilitate bone fusion between two or more adjacent vertebrae.

One procedure for treating spinal disorders involves attaching a rigid fixation system of bone screws and rods to the posterior side of the vertebrae to position the vertebrae for bone fusion. A patient may be positioned to permit surgical access to the spinal area. Once the spine is exposed, the vertebrae may be instrumented with a series of screw assemblies that are driven into each of the vertebrae for attachment of fixation rods. The screw assemblies may include mono-axial or poly-axial screws that are driven into the pedicles of the vertebrae. The screw assemblies also include rod receiving heads that couple with the screws. The heads typically include threaded channels for receiving the fixation rods.

The fixation rods may be shaped to a predetermined alignment and curvature depending on the anatomy of the patient. The fixation rods are inserted into the threaded channels of the heads. Locking set screws are inserted into the threaded channels and tightened to secure the fixation rods and screws together within the heads. Prior to or after fixation of the vertebrae, a spinal spacer, bone graft, and/or bone material may be inserted between two or more instrumented vertebrae to fuse the bones together. Thus, the screw assemblies and fixation rods hold the vertebrae in position to improve fusion of the vertebrae during patent recovery periods. Examples of screw assemblies can be seen in EP 2,022,423, EP 2,153,786 and WO 2006/089292.

Typically, spinal fixation systems, including fixation rods and screw assemblies, are designed to be substantially rigid and allow little or no movement of the instrumented, fixed vertebrae. Thus, inter-vertebral movement between any of the fixed vertebrae is substantially limited. As a patient begins to move during recovery, stresses and forces that would normally act on the fixed vertebrae may be transferred by the rigid fixation system to adjacent, non-instrumented vertebrae. This type of rigid fixation system may be preferred for a variety of reasons. For example, rigid fixation may be preferred to alleviate stress on nearby nerves, tissue, and/or a damaged vertebra. Rigid fixation may be preferred to prevent disruption of the fusion process between fixed vertebrae due to movement of an implant, bone graft, or bone cement when the patient begins to move during recovery.

In addition to these benefits, this type of rigid fixation may also negatively impact patient recovery in a number of ways. For example, according to the principle of Wolffs law, bone growth may be accelerated when stress is applied to targeted areas of the healing bones. Therefore, vertebrae that are deprived of stress may be less apt to successfully fuse with adjacent bone segments as desired. Also, non-instrumented vertebrae above and below the instrumented vertebrae must absorb the loads that would ordinarily be shared by the instrumented vertebrae. These non-instrumented vertebrae also must provide a greater range of motion to compensate for the lost range of motion of the instrumented vertebrae. Last, in a typical rigid construct, the weakest point in the fixation system is located at the vertebra/screw intersection. This may result in screw loosening since bone material inside the vertebra, which is a low rigidity material, absorbs most of the stresses.

### SUMMARY

A semi-rigid head for a bone screw comprising includes a first rigid portion, a second rigid portion, and a semi-rigid portion. The first rigid portion is configured to receive a fixation rod. The second rigid portion is configured for coupling with a bone screw. The semi-rigid portion links the first rigid portion and the second rigid portion.

In other features, the semi-rigid portion includes flexible members that link the first rigid portion and the second rigid portion. The semi-rigid portion controls movement of the first rigid portion relative to the second rigid portion in a direction substantially parallel to the fixation rod. The semi-rigid portion permits a predetermined amount of movement of the first rigid portion relative to the second rigid portion. The first rigid portion includes a top portion, the second rigid portion includes a bottom portion opposite the top portion, and the semi-rigid portion includes four flexible members linking the top portion and the bottom portion. The semi-rigid portion includes joints comprising at least one of pivoting hinges, springs, bushings, varying material properties, and living hinges. The first rigid portion includes a saddle that extends distally inside a cavity of the semi-rigid head. The second rigid portion includes a collet that extends distally for coupling with a driving end of the bone screw.

In yet other features, a locking member with a bottom portion having an aperture slides over the collet to rigidly couple the semi-rigid head to the driving end. The locking member includes side portions extending proximally from the bottom portion to engage with a set screw.

A polyaxial screw assembly includes a bone screw, a semi-rigid head, a locking member, and a set screw. The bone screw includes a threaded end for attachment to a vertebra and a driving end. The semi-rigid head includes a first rigid portion that receives a fixation rod, a second rigid portion that couples to the driving end, and a semi-rigid portion linking the first and second rigid portions. The locking member includes a locked position and an unlocked position. The set screw selectively engages the fixation rod to lock the fixation rod within the first rigid portion and selectively positions the locking member to lock the second rigid portion to the driving end in the locked position. The semi-rigid portion allows a predetermined amount of movement of the fixation rod relative to the vertebra when the locking member is in the locked position.

In other features, the semi-rigid head rotates freely about the driving end when the locking member is in the unlocked position. The set screw includes a first portion that engages the fixation rod and a second portion that engages the locking member. The first rigid portion includes a saddle that receives the fixation rod. The second rigid portion includes a collet that couples to the driving end. The locking member includes an aperture that compresses the collet in the locked position.

A method of securing ; (not claimed) a fixation rod to a bone screw with a polyaxial head includes the step of inserting the fixation rod within a first rigid portion of the head. The method includes coupling a second rigid portion of the head to a driving end of the bone screw, wherein the first and second rigid portions are linked by a semi-rigid portion. The method includes coupling a locking member to the second rigid portion, the locking member positionable between a locked position that locks the second rigid portion to the driving end and an unlocked position that permits polyaxial movement of the head relative to the driving end. The method includes inserting a set screw within the first rigid portion to selectively lock the fixation rod within the first rigid portion and to selectively position the locking member between the locked position and the unlocked position.

In other examples coupling the locking member to the first rigid portion includes inserting a collet of first rigid portion through an aperture of the locking member. The method (not claimed) advancing the set screw until a first end engages the fixation rod and locks the fixation rod to the first rigid portion. The method includes continuing to advance the set screw until a second end engages the locking member to position the aperture and compress the collet to lock the first rigid portion to the driving end.

### BRIEF DESCRIPTION OF THE DRAWINGS

FIG. 1 is a perspective view of the semi-rigid screw assembly according to the principles of the present disclosure coupled with a rigid fixation rod.
FIG. 2 is a perspective view of a series of semi-rigid screw assemblies according to the principles of the present disclosure and a rigid fixation rod attached to a spinal column.
FIG. 3 is an exploded perspective view of the semi-rigid screw assembly in a polyaxial screw configuration according to the principles of the present disclosure.
FIG. 4 is an exploded perspective view of another semi-rigid screw assembly in a mono-axial screw configuration according to the principles of the present disclosure.
FIG. 5 is a perspective view of a semi-rigid head of the semi-rigid screw assembly according to the principles of the present disclosure.
FIG. 6 is an elevational front view of the semi-rigid head according to the principles of the present disclosure.
FIG. 7 is an elevational side view of the semi-rigid screw according to the principles of the present disclosure in a natural, rest position.
FIG. 8 is an elevational side view of the semi-rigid screw according to the principles of the present disclosure in a deformed position.
FIG. 9 is an elevational front view of the semi-rigid screw assembly and a fixation rod in an unlocked position.
FIG. 10 is an elevational front view of the semi-rigid screw assembly and a fixation rod in a locked position.

### DETAILED DESCRIPTION

A semi-rigid screw assembly according to the principles of the present disclosure improves patient recovery in a number of ways. The semi-rigid screw assembly allows a predetermined amount of controlled movement between instrumented vertebrae to promote bone fusion according to Wolff's law. The screw assembly also evenly distributes loads among the instrumented vertebrae and provides improved range of motion of the instrumented vertebrae. The stress distribution within the instrumented segment of the vertebrae is better distributed among several areas of the fixation system. By creating a flexible or semi-rigid region in the screw, stresses may be dissipated among multiple screws and vertebrae, thus decreasing the stress on the bone marrow and preserving structure that supports the bone screws.

The semi-rigid screw assembly of the present disclosure provides a semi-rigid attachment of a rigid fixation rod to the screw assembly. The semi-rigid screw assembly allows a calibrated amount of controlled movement of the fixation rod relative to the screw assembly. Thus, when two or more vertebrae are instrumented, some movement between the instrumented vertebrae may occur. The semi-rigid screw assembly may include a number of configurations including a poly-axial configuration and a mono-axial configuration.

In a poly-axial configuration, the semi-rigid screw assembly includes a bone screw that attaches to the vertebra and a semi-rigid head that couples with the driving end of the bone screw. In a mono-axial configuration, the semi-rigid head may be integral with the bone screw. The semi-rigid head includes a first rigid portion that couples with the bone screw and a second rigid portion for attachment of the fixation rod. A semi-rigid portion between the first and second rigid portions allows the second rigid portion to move relative to the first rigid portion. When the fixation rod is attached to the second portion, the fixation rod may move relative to the first portion. Thus, the semi-rigid head allows a calibrated amount of controlled movement between two or more instrumented vertebrae to improve bone fusion and reduce stress on adjacent non-instrumented vertebrae.

Embodiments of the invention will now be described with reference to the Figures, wherein like numerals reflect like elements throughout. The terminology used in the description presented herein is not intended to be interpreted in any limited or restrictive way, simply because it is being utilized in conjunction with detailed description of certain specific embodiments of the invention. Furthermore, embodiments of the invention may include several novel features, no single one of which is solely responsible for its desirable attributes or which is essential to practicing the invention described herein. The words proximal and distal are applied herein to denote specific ends of components of the instrument described herein. A proximal end refers to the end of an instrument nearer to an operator of the instrument when the instrument is being used. A distal end refers to the end of a component further from the operator and extending towards the surgical area of a patient and/or the implant.

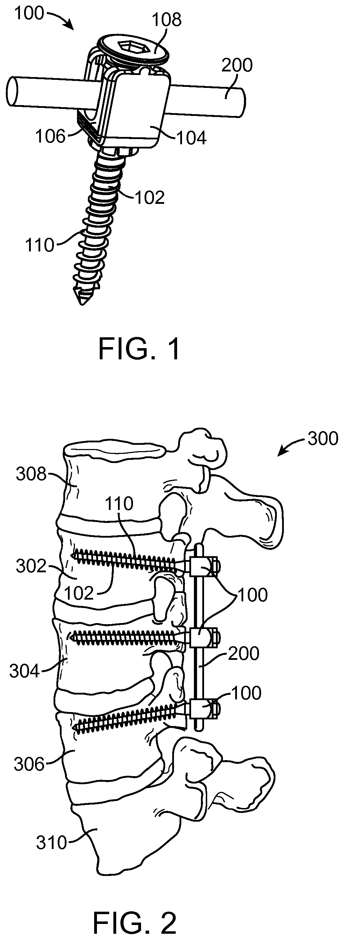

Referring now to FIG. 1, a semi-rigid screw assembly (hereinafter "the screw assembly") 100 according to the principles of the present disclosure is shown with a fixation rod 200 attached in a poly-axial screw configuration. The screw assembly 100 may include a bone screw 102, a locking member 104, a semi-rigid head 106 for receiving the fixation rod 200, and a flanged screw 108 in the poly-axial screw configuration. The bone screw 102 includes a threaded end 110 for attachment to a spinal column 300 as shown in FIG. 2. In FIG. 2, the spinal column 300 has been instrumented with multiple screw assemblies 100 and the fixation rod 200. Each screw assembly 100 may be attached to one of several vertebrae 302, 304, and 306 by driving the threaded end 110 into the vertebrae. Once the vertebrae 302-306 are instrumented with the screw assemblies 100, the fixation rod 200 may be attached. Adjacent vertebra 308 and 310 may not be instrumented with screw assemblies.

The semi-rigid head 106 may include rigid portions and semi-rigid portions that allow a predetermined amount of controlled movement of the fixation rod 200. The amount of movement allowed may be based on factors that include preventing disruption of bone fusion while also encouraging bone fusion according to Wolff's Law. The screw assemblies 100 may allow movement in one or more directions. For example, with respect to FIG. 2, the screw assemblies 100 may allow movement that is substantially parallel to the spinal column 300 and that allows a small amount of compression and decompression in the space between the instrumented vertebrae 302-306. This compression and decompression may encourage bone fusion between the instrumented vertebrae 302-306 while reducing stress on the adjacent vertebrae 308 and 310.

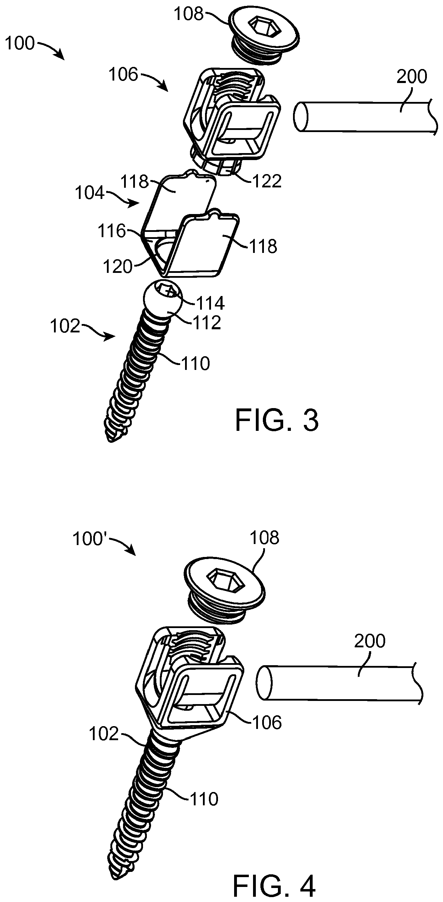

Referring now to FIG. 3, an exploded view of the screw assembly 100 illustrates additional features of the bone screw 102, the locking member 104, and the semi-rigid head 106. The bone screw 102 includes a driving end 112 at a proximal end that couples with the semi-rigid head 106. A driving feature 114 of the driving end 112, such as a star- or hex-head configuration, may be driven by a screwdriver to advance the threaded end 110 further into the vertebrae. The locking member 104 includes a bottom portion 116 and side portions 118 that form a substantially U-shaped collar that slides around the semi-rigid head 106. As the locking member 104 slides around the semi-rigid head 106, an aperture 120 in the locking member 104 receives an expandable collet 122 of the semi-rigid head 106. Although the present example refers to a poly-axial screw configuration, one skilled in the art may appreciate that in other configurations, such as a mono-axial screw configuration, the screw assembly 100' may include an integral bone screw 102 and semi-rigid head 106 as shown in FIG. 4. Thus, for example, in the mono-axial screw configuration, the locking member 104 and collet 122 may not be necessary to lock the semi-rigid head 106 to the bone screw 102.

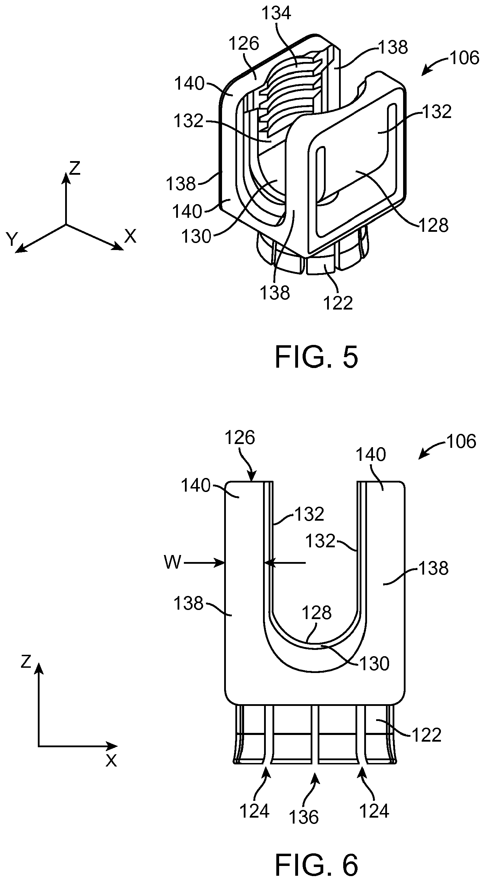

Continuing now with FIGS. 5-8, additional features of the semi-rigid head 106 may be described with reference to orthogonal axes X, Y, and Z. The semi-rigid head 106 includes substantially rigid portions at proximal and distal ends that are linked together by flexible or semi-rigid members. The proximal end of the semi-rigid head 106 includes a top portion 126 configured to receive the fixation rod 200. For example, the top portion 126 may include a saddle 128 that extends into an interior cavity of the semi-rigid head 106. The saddle 128 may be a U-shaped sleeve with a curved portion 130 at a distal end having curvature corresponding to a diameter of the fixation rod 200. Two side portions 132 extend from the curved portion 130 towards the proximal end of the semi-rigid head 106 to form the top portion 126. Threads 134 in the side portions 132 are configured to mate with the flanged screw 108 and enable rigid attachment of the fixation rod 200 to the saddle 128.

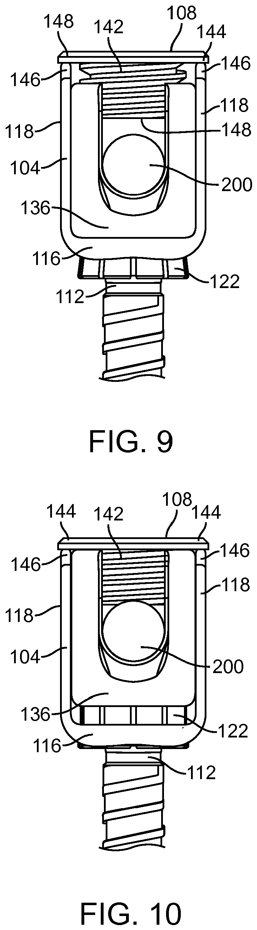

The distal end of the semi-rigid head 106 includes a bottom portion 136 that couples with the bone screw 102. In the poly-axial configuration of FIGS. 3 and 5-8, the bottom portion 136 may include the expandable collet 122 for attachment to the driving end 112 of the bone screw 102. The collet 122 and the locking member 104 may be used to rigidly couple the semi-rigid head 106 to the driving end 112 of the bone screw 102. The collet 122 may include a number of kerf cuts 124 that enable elastic and/or plastic expansion and contraction of the collet 122. For example, the collet 122 may contract when inserted through the aperture 120 of the locking member 104 and expand after passing therethrough. The collet 122 may contract around the driving end 112 of the bone screw 102 when the locking member 104 is actuated as described with reference to FIGS. 9 and 10. In the mono-axial screw configuration of FIG. 4, the bottom portion 136 may be integral with the bone screw 102. Thus, in the poly-axial and mono-axial screw configurations, the bottom portion 136 may be rigidly coupled to or integral with the bone screw 102.

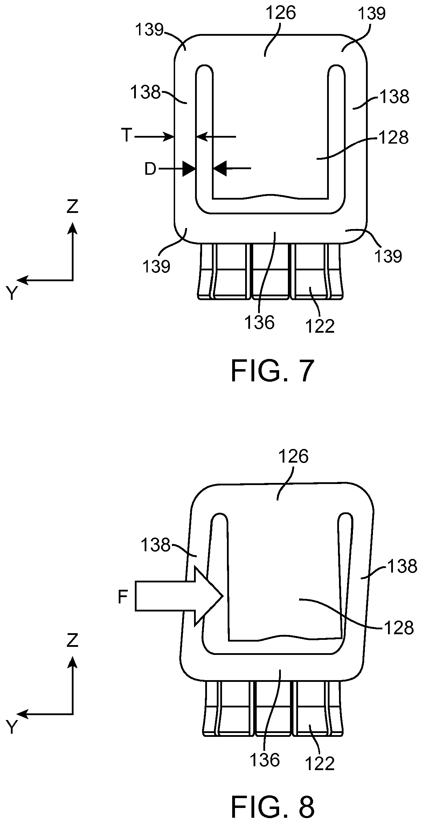

The semi-rigid head 106 further includes semi-rigid portions 138 disposed between the top portion 126 and the bottom portion 136 to enable a predetermined amount of controlled movement of the fixation rod 200 relative to the screw assemblies 100. The semi-rigid portions 138 may be elastically flexible. For example, the semi-rigid portions 138 may include four legs 138 that connect the top portion 126 and the bottom portion 136 to substantially form a cuboidal geometry. The legs 138 may include joints 139 where connected to the top portion 126 and bottom portion 136. Each leg 138 includes a width W and a thickness T that determine the rigidity of the leg 138 and thus, the amount of movement each leg 138 may allow based on a predetermined level of force F. For example, in FIGS. 6 and 7, the width W is greater than the thickness T which permits substantially more movement in the Y-direction than in the X-direction. In this configuration, as shown in FIG. 2, the semi-rigid connection between the fixation rod 200 and the bone screw 102 allows greater compression and decompression of the instrumented vertebrae 302, 304, and 306 when compared with a traditional rigid fixation system.

In other examples, each leg 138 may include a varying width W and thickness T that determine a varying amount of rigidity along the length of each leg 138. For example, where the legs 138 join the top portion 126 and the bottom portion 136, the thickness T may be less than the thickness T near the center of the legs 138. In other examples, the legs 138 and joints 139 may include additional features, such as pivoting hinges, springs, bushings, varying material properties, living hinges, and the like, that allow a predetermined amount of controlled movement of the top portion 126 relative to the bottom portion 136. In still other examples, the amount of controlled movement permitted may be adjusted by varying a distance D between the legs 138 and the saddle 128. For example, the greater the distance D is, the greater the amount of permitted movement before the saddle 128 begins to contact one of the legs 138. The smaller the distance D is, the lesser the amount of permitted movement before the saddle 128 begins to contact one of the legs 138.

By varying any of the dimensions W, T, and D of the semi-rigid portions 138 of the semi-rigid head 106, the rigidity and amount of predetermined movement may be adjusted to conform to any particular surgical and recovery requirements. Thus, the semi-rigid head 106 may be configured for a variety of patient characteristics and surgical locations. For example, different areas of the spine support different amounts of weight based on the location along the spinal column and/or the anatomy of the patient. When lower weights are supported, the semi-rigid portions 138 may include dimensions that require less force F to move than when higher weights are supported. For example, in the cervical region of the spine, less weight is supported by those vertebrae than by vertebrae in the thoracic region of the spine. Thus, the dimensions of the semi-rigid head 106 may be smaller for cervical applications than for thoracic applications.

Although the terms rigid and semi-rigid are used throughout the present disclosure, one skilled in the art may understand that rigidity may also be describe in terms of an amount of flexibility. Thus, for example, by varying the dimensions of the semi-rigid portions 138 of the semi-rigid head 106, the flexibility may be adjusted. The more flexible the semi-rigid portions 138 are, the less force required to deform the semi-rigid head 106. The less flexible the semi-rigid portions 138 are, the more force that is required to deform the semi-rigid head 106.

Referring now to FIGS. 7 and 8, movement of the top portion 126 including the saddle 128 relative to the bottom portion 136 occurs as the semi-rigid portions 138 of the semi-rigid head 106 elastically deform when force F is applied. For example, at rest as shown in FIG. 7, the legs 138 are substantially parallel to the Z-axis. When the force F is applied in the Y-direction, or substantially parallel with the spinal column in FIG. 2, the semi-rigid head 106 may bend or flex substantially in the Y-direction. In this configuration, the legs 138 permit compression and decompression of the space between the attached instrumented vertebrae 302-306. As stated above, the dimensions of the legs 138 may be configured to allow a predetermined amount of movement based on the force F. The legs 138 may also be configured to provide controlled movement along a predetermined path or direction. For example, the legs 138 may provide controlled movement along an axial path corresponding to the longitudinal axis of the fixation rod 200. Thus, the semi-rigid head 106 may elastically deform from a natural, rest position in FIG. 7 to a deformed position in FIG. 8.

Although the semi-rigid head 106 of the present example provides flexibility in one direction, multiple directions of movement may be allowed for various applications. By varying the dimensions of the semi-rigid portions 138 of the semi-rigid head 106, the direction of the controlled movement may also be adjusted to conform to any particular surgical and recovery requirements. Thus, the semi-rigid head 106 may be configured for a variety of patient characteristics and surgical locations. For example, different areas of the spine may require movement in one or more directions corresponding to the X, Y, and Z axes. In lower regions of the spine, such as the lumbar region, less twisting of the spine may occur than in higher regions of the spine such as the thoracic region. Thus, the semi-rigid head 106 may be configured to allow for movement in two directions in the thoracic region and one direction in the lumbar region.

Referring now to FIGS. 9 and 10, actuation of the locking member 104 and collet 122 of the poly-axial configuration are illustrated in greater detail. In FIG. 9, the side portions 118 of the locking member 104 slide around the semi-rigid head 106. The collet 122 of the semi-rigid head 106 passes through the aperture 120 (not shown) in the bottom portion 116 of the locking member 104 and couples with the driving end 112 of the bone screw 102. The driving end 112 may include a rounded portion for us in a poly-axial screw configuration. Threads 142 of the flanged screw 108 begin to engage with the mating threads 134 (not shown) of the semi-rigid head 106. A portion of the flanged screw 108, such as an outer edge 144, begins to engage with the side portions 118 of the locking member 104. For example, the side portions 118 may include projections 146 that extend away from the proximal ends of the side portions 118 to engage with the outer edge 144.

As the flanged screw 108 is tightened, more of the threads 142 engage the threads 134 of the semi-rigid head 106. A distal end 148 of the flanged screw 108 compresses the fixation rod 200 against the saddle 128. Simultaneously, the outer edge 144 of the flanged screw 108 may push down on the projections 146, forcing the bottom portion 116 of the locking member 104 away from the bottom portion 136 of the semi-rigid head 106. As the aperture 120 (not shown) in the bottom portion 116 passes over the collet 122, the collet 122 contracts around the driving end 112 of the bone screw 102. As the flanged screw 108 is fully tightened, the distal end 148 rigidly couples the fixation rod 200 within the saddle 128 and the locking member 104 rigidly couples the collet 122 with the driving end 112 of the bone screw 102.

Although a single flanged screw 108 is illustrated in the current example screw assembly 100, multiple screws may be used to compress the fixation rod 200 against the saddle and force the bottom portion 116 of the locking member 104 to compress the collet 122 around the driving end 112. For example a pair of concentric screws may be used. The flanged screw 108 may include a concentric set screw (not shown) that may be tightened downwardly to compresses the fixation rod 200 against the saddle 128. The flanged screw 108 may be tightened separately from this set screw to rigidly lock the semi-rigid head 106 with the driving end 112.

Example embodiments of the semi-rigid heads and polyaxial screw assemblies of the present invention have been described herein. As noted elsewhere, these example embodiments have been described for illustrative purposes only, and are not limiting. Other embodiments are possible and are covered by the invention. Such embodiments will be apparent to persons skilled in the relevant art(s) based on the teachings contained herein. Thus, the breadth and scope of the present invention should not be limited by any of the above-described exemplary embodiments, but should be defined only in accordance with the following claims.

## Claims

1. A semi-rigid head (106) for a bone screw **characterized by** comprising:
a first rigid portion (126) configured to receive a fixation rod (200);
a second rigid portion (136) configured for coupling with a bone screw (102);
a semi-rigid portion (138) linking the first rigid portion (126) with the second rigid portion (136); and wherein the first rigid portion (126) includes a saddle (128) that extends distally inside a cavity of the semi-rigid head (106).

2. The semi-rigid head of claim 1, wherein the semi-rigid portion (138) includes flexible members that link the first rigid portion and the second rigid portion.

3. The semi-rigid head of claim 1, wherein the semi-rigid portion (138) controls movement of the first rigid portion (126) relative to the second rigid portion (136), preferably when a fixation rod (200) is received within the first rigid portion then the control of movement is in a direction substantially parallel to the fixation rod (200).

4. The semi-rigid head of claim 1, wherein the semi-rigid portion (138) permits a predetermined amount of movement of the first rigid portion (126) relative to the second rigid portion (136).

5. The semi-rigid head of claim 1, wherein the first rigid portion (126) includes a top portion, the second rigid portion (136) includes a bottom portion opposite the top portion, and the semi-rigid portion (138) includes four flexible members (138) linking the top portion and the bottom portion.

6. The semi-rigid head of claim 1, wherein the semi-rigid portion includes joints (139) comprising at least one of pivoting hinges, springs, bushings, varying material properties, and living hinges.

7. The semi-rigid head of claim 1, wherein the second rigid portion (136) includes a collet (122) that extends distally for coupling with a driving end of the bone screw (102).

8. The semi-rigid head of claim 7, further comprising a locking member (104) with a bottom portion (116) having an aperture (120) that slides over the collet (122) to rigidly couple the semi-rigid head (106) to the driving end (112), and preferably wherein the locking member (104) includes side portions (118) extending proximally from the bottom portion (116) to engage with a set screw.

9. A polyaxial screw assembly (100) comprising:
a semi-rigid head (106) of claim 1;
a bone screw (102) that includes a threaded end (110) for attachment to a vertebra (302, 304, 306) and a driving end (112);
a locking member (104) having a locked position and an unlocked position; and
a set screw (108) that selectively engages the fixation rod (200) to lock the fixation rod (200) within the first rigid portion (130) and selectively positions the locking member (104) to lock the second rigid portion to the driving end (112) in the locked position,
wherein the semi-rigid portion allows a predetermined amount of movement of the fixation rod (200) relative to the vertebra when the locking member (104) is in the locked position.

10. The polyaxial screw assembly of claim 9, wherein the semi-rigid head rotates freely about the driving end when the locking member is in the unlocked position.

11. The polyaxial screw assembly of claim 9, wherein the set screw includes a first portion that engages the fixation rod and a second portion that engages the locking member.

12. The polyaxial screw assembly of claim 9, wherein the first rigid portion includes a saddle that receives the fixation rod.

13. The polyaxial screw assembly of claim 9, wherein the second rigid portion includes a collet that couples to the driving end, and preferably wherein the locking member includes an aperture that compresses the collet in the locked position.

## Patentansprüche

1. Halbstarrer Kopf (106) für eine Knochenschraube, **dadurch gekennzeichnet, dass** sie Folgendes umfasst:
einen ersten starren Abschnitt (126), der dazu ausgebildet ist, einen Fixierstab (200) aufzunehmen;
einen zweiten starren Abschnitt (136), der zur Kopplung mit einer Knochenschraube (102) ausgebildet ist;
einen halbstarren Abschnitt (138), der den ersten starren Abschnitt (126) mit dem zweiten starren Abschnitt (136) verbindet; und wobei der erste starre Abschnitt (126) einen Sattel (128) aufweist, der sich distal innerhalb eines Hohlraums des halbstarren Kopfes (106) erstreckt.

2. Halbstarrer Kopf nach Anspruch 1, wobei der halbstarre Abschnitt (138) flexible Glieder aufweist, die den ersten starren Abschnitt und den zweiten starren Abschnitt verbinden.

3. Halbstarrer Kopf nach Anspruch 1, wobei der halbstarre Abschnitt (138) eine Bewegung des ersten starren Abschnitts (126) bezogen auf den zweiten starren Abschnitt (136) steuert, vorzugsweise wenn ein Fixierstab (200) innerhalb des ersten starren Abschnitts aufgenommen ist, die Bewegungssteuerung dann in einer Richtung im Wesentlichen parallel zum Fixierstab (200) erfolgt.

4. Halbstarrer Kopf nach Anspruch 1, wobei der halbstarre Abschnitt (138) eine vorbestimmte Bewegungsmenge des ersten starren Abschnitts (126) bezogen auf den zweiten starren Abschnitt (136) gestattet.

5. Halbstarrer Kopf nach Anspruch 1, wobei der erste starre Abschnitt (126) einen oberen Abschnitt aufweist, der zweite starre Abschnitt (136) einen unteren Abschnitt gegenüber dem oberen Abschnitt aufweist und der halbstarre Abschnitt (138) vier flexible Glieder (138) aufweist, die den oberen Abschnitt und den unteren Abschnitt verbinden.

6. Halbstarrer Kopf nach Anspruch 1, wobei der halbstarre Abschnitt Verbindungen (139) aufweist, die mindestens eins von Schwenkscharnieren, Federn, Buchsen, variierenden Materialeigenschaften und Filmscharnieren umfassen.

7. Halbstarrer Kopf nach Anspruch 1, wobei der zweite starre Abschnitt (136) eine Klemmbuchse (122) aufweist, die sich zur Kopplung mit einem Triebende der Knochenschraube (102) distal erstreckt.

8. Halbstarrer Kopf nach Anspruch 7, ferner umfassend ein Verriegelungselement (104) mit einem unteren Abschnitt (116), der eine Öffnung (120) aufweist, die über die Klemmbuchse (122) gleitet, um den halbstarren Kopf (106) starr an das Triebende (112) zu koppeln, und wobei vorzugsweise das Verriegelungselement (104) Seitenabschnitte (118) aufweist, die sich proximal von dem unteren Abschnitt (116) erstrecken, um mit einer Stellschraube in Eingriff zu treten.

9. Polyaxiale Schraubenanordnung (100) umfassend:
einen halbstarren Kopf (106) nach Anspruch 1;
eine Knochenschraube (102), die ein Gewindeende (110) zur Anbringung an einem Wirbel (302, 304, 306) und ein Triebende (112) aufweist;
ein Verriegelungselement (104) mit einer verriegelten Position und einer entriegelten Position; und
eine Stellschraube (108), die selektiv mit dem Fixierstab (200) in Eingriff tritt, um den Fixierstab (200) innerhalb des ersten starren Abschnitts (130) zu verriegeln, und das Verriegelungselement (104) selektiv positioniert, um den zweiten starren Abschnitt mit dem Triebende (112) in der verriegelten Position zu verriegeln,
wobei der halbstarre Abschnitt eine vorbestimmte Bewegungsmenge des Fixierstabs (200) bezogen auf den Wirbel gestattet, wenn sich das Verriegelungselement (104) in der verriegelten Position befindet.

10. Polyaxiale Schraubenanordnung nach Anspruch 9, wobei sich der halbstarre Kopf frei um das Triebende dreht, wenn sich das Verriegelungselement in der entriegelten Position befindet.

11. Polyaxiale Schraubenanordnung nach Anspruch 9, wobei die Stellschraube einen ersten Abschnitt aufweist, der mit dem Fixierstab in Eingriff tritt, und einen zweiten Abschnitt, der mit dem Verriegelungselement in Eingriff tritt.

12. Polyaxiale Schraubenanordnung nach Anspruch 9, wobei der erste starre Abschnitt einen Sattel aufweist, der den Fixierstab aufnimmt.

13. Polyaxiale Schraubenanordnung nach Anspruch 9, wobei der zweite starre Abschnitt eine Klemmbuchse aufweist, die mit dem Triebende gekoppelt ist, und wobei vorzugsweise das Verriegelungselement eine Öffnung aufweist, die die Klemmbuchse in der verriegelten Position zusammendrückt.

## Revendications

1. Une tête semi-rigide (106) pour une vis à os **caractérisée en ce qu'**elle comprend :
une première partie rigide (126) configurée pour recevoir une tige de fixation (200) ;
une deuxième partie rigide (136) configurée pour s'accoupler avec une vis à os (102) ;
une partie semi-rigide (138) reliant la première partie rigide (126) à la deuxième partie rigide (136) ; et dans laquelle la première partie rigide (126) comprend une selle (128) qui s'étend de manière distale à l'intérieur d'une cavité de la tête semi-rigide (106).

2. La tête semi-rigide selon la revendication 1, dans laquelle la partie semi-rigide (138) comprend des éléments flexibles qui relient la première partie rigide et la deuxième partie rigide.

3. La tête semi-rigide selon la revendication 1, dans laquelle la partie semi-rigide (138) contrôle le mouvement de la première partie rigide (126) par rapport à la deuxième partie rigide (136), de préférence lorsqu'une tige de fixation (200) est reçue à l'intérieur de la première partie rigide, alors le contrôle du mouvement est dans une direction sensiblement parallèle à la tige de fixation (200).

4. La tête semi-rigide selon la revendication 1, dans laquelle la partie semi-rigide (138) permet une quantité prédéterminée de mouvement de la première partie rigide (126) par rapport à la deuxième partie rigide (136).

5. La tête semi-rigide selon la revendication 1, dans laquelle la première partie rigide (126) comprend une partie supérieure, la deuxième partie rigide (136) comprend une partie inférieure opposée à la partie supérieure, et la partie semi-rigide (138) comprend quatre éléments flexibles (138) reliant la partie supérieure et la partie inférieure.

6. La tête semi-rigide selon la revendication 1, dans laquelle la partie semi-rigide comprend des articulations (139) comprenant au moins un parmi des charnières pivotantes, des ressorts, des coussinets, des propriétés de matériau variables, et des charnières vivantes.

7. La tête semi-rigide selon la revendication 1, dans laquelle la deuxième partie rigide (136) comprend une pince de serrage (122) qui s'étend de manière distale pour s'accoupler avec une extrémité d'entraînement de la vis à os (102).

8. La tête semi-rigide selon la revendication 7, comprenant en outre un élément de verrouillage (104) avec une partie inférieure (116) ayant une ouverture (120) qui coulisse sur la pince de serrage (122) pour coupler de manière rigide la tête semi-rigide (106) à l'extrémité d'entraînement (112), et de préférence dans laquelle l'élément de verrouillage (104) comprend des parties latérales (118) s'étendant de manière proximale depuis la partie inférieure (116) pour s'engager avec une vis de blocage.

9. Un ensemble de vis polyaxiales (100) comprenant :
une tête semi-rigide (106) selon la revendication 1 ;
une vis à os (102) qui comprend une extrémité filetée (110) pour la fixation à une vertèbre (302, 304, 306) et une extrémité d'entraînement (112) ;
un élément de verrouillage (104) ayant une position verrouillée et une position déverrouillée ; et
une vis de blocage (108) qui engage sélectivement la tige de fixation (200) pour verrouiller la tige de fixation (200) à l'intérieur de la première partie rigide (130) et positionne sélectivement l'élément de verrouillage (104) pour verrouiller la deuxième partie rigide à l'extrémité d'entraînement (112) dans la position verrouillée,
dans lequel la partie semi-rigide permet une quantité prédéterminée de mouvement de la tige de fixation (200) par rapport à la vertèbre lorsque l'élément de verrouillage (104) est dans la position verrouillée.

10. L'ensemble de vis polyaxiales selon la revendication 9, dans lequel la tête semi-rigide tourne librement autour de l'extrémité d'entraînement lorsque l'élément de verrouillage est dans la position déverrouillée.

11. L'ensemble de vis polyaxiales selon la revendication 9, dans lequel la vis de blocage comprend une première partie qui engage la tige de fixation et une deuxième partie qui engage l'élément de verrouillage.

12. L'ensemble de vis polyaxiales selon la revendication 9, dans lequel la première partie rigide comprend une selle qui reçoit la tige de fixation.

13. L'ensemble de vis polyaxiales selon la revendication 9, dans lequel la deuxième partie rigide comprend une pince de serrage qui s'accouple à l'extrémité d'entraînement, et de préférence dans lequel l'élément de verrouillage comprend une ouverture qui comprime la pince de serrage dans la position verrouillée.
